# EUROPEAN PATENT APPLICATION

(11) **EP 1 935 441 A1**
(43) Date of publication of application: **25.06.2008**
(21) Application number: 07101127.4
(22) Date of filing: 24.01.2007
(51) Int. Cl.: A61M 1/16, A61M 1/34

(54) **Device for the removal of toxic substances from blood**

(30) Priority: 21.12.2006 EP 06077295
(71) Applicant: Nederlandse Organisatie voor Toegepast-Natuurwetenschappelijk Onderzoek TNO, 2628 VK Delft (NL)
(72) Inventor: Schilthuizen, Stephanus Franciscus, 5056 MG Berkel-Enschot (NL); Batenburg, Lawrence Fabian, 5652 EJ Eindhoven (NL); Simonis, Frank, 5688 RX Oirschot (NL); Vercauteren, Franky Flory, 5644 DK Eindhoven (NL)
(74) Representative: van Loon, C.J.J.

(57) **Abstract**

The present invention relates to a device for the removal of toxic substances from dialysate fluids and/or from blood from a patient comprising a sorption-filter device (2) for removing toxins, small and middle-sized molecules from dialysate fluids and/or patient blood plasma, said sorption filter comprising as a sorbing material a smectite, nanoclay, a layered double hydroxide, a modified biopolymer or a combination thereof.

## Description

The present invention is in the field of artificial kidneys and dialysis systems. The invention relates to a device for the removal of toxic substances from blood from a patient, to methods of removing toxic substances from blood using the inventive device and to filter pads suitable for use in said device and in hemodialysis and peritoneal dialysis systems.

Hemodialysis (HD) and peritoneal dialyis (PD) are methods of removing toxic substances (impurities or wastes) from the blood when the kidneys are unable to do so sufficiently. Dialysis is most frequently used for patients who have kidney failure, but may also be used to quickly remove drugs or poisons in acute situations. This technique can be life saving in people with acute or chronic kidney failure. Best known is hemodialysis, which works by circulating the blood along special filters outside the body in a dialysis machine. Here, the blood flows across a semi-permeable membrane (the dialyser or filter), on the other side of which flows a dialysis fluid in a counter-current direction to the blood flow. The dialysing membrane allows passage of substances below a certain molecular cut-off. By diffusion the concentration of these substances will end up being the same on both sides of the membrane. The dialysis fluid removes the toxins from the blood and is generally discarded as waste dialysate. The chemical imbalances and impurities of the blood are being brought back in minimal balance and the blood is then returned to the body. The efficacy of hemodialysis is approximately 10%, which means that 10% of the toxins are being removed from the blood. Typically, most patients undergo hemodialysis for three sessions every week. Each session lasts normally 3-4 hours. This is very inconvenient, and the physical and social side effects of dialysis to the patients are a great concern.

The efficacy of peritoneal dialysis is even lower. In PD, a soft catheter is used to fill the abdomen with a dialysis solution that generally contains dextrose. The peritoneal membrane, which lines the walls of the abdominal cavity, allows waste products and extra fluid to pass from the blood into the dialysis solution. The saturated dialysis solution is then drained from the body and is discarded. The exchange process of filling and draining takes about 30 to 40 minutes.

In order to provide for portable dialysis devices, that will allow patients to engage in normal daily activities, artificial kidneys have been developed. Essentially there are two types of artificial kidneys.

In one form, the principle of the artificial kidney consists of extracting urea and other more toxic middle molecules from blood by dialysis and regeneration of the dialysate by means of an adsorbent, usually activated carbon. In the case of a system based on such a dialysis kidney machine, a key aspect resides in regenerating the dialysis fluid when the latter is to be recycled into the dialyser. Dialysis kidney machines that can be encountered in the prior art include for instance those described in GB 1 406 133, and US 2003/0097086. GB 1 406 133 discloses an artificial kidney of the recycle type having an improved adsorbent comprising activated carbon and alumina. US 2003/0097086 discloses a portable dialysis device comprising dialyzers connected in series that utilize dialysate, and further comprising a plurality of contoured sorbent devices, which are connected in series and are for regenerating the spent dialysate. As adsorption materials for regeneration of the spent dialysate, activated charcoal, urease, zirconium phosphate, hydrous zirconium oxide and/or activated carbon are provided.

In another form, the principle of the artificial kidney may be based on ultrafiltration, or hemofiltration, using appropriate membranes, wherein large molecules including blood cells are retained in the retentate on the filter, and the toxic substances are collected in the (ultra)filtrate. During hemofiltration, a patient's blood is passed through a set of tubing (a filtration circuit) via a machine to a semipermeable membrane (the filter) where waste products and water are removed. Replacement fluid is added and the blood is returned to the patient. In a similar fashion to dialysis, hemofiltration involves the movement of solutes across a semi-permeable membrane. However, the membrane used in hemofiltration is far more porous than that used in hemodialysis, and no dialysate is used-instead a positive hydrostatic pressure drives water and solutes across the filter membrane where they are drained away as filtrate. An isotonic replacement fluid is added to the resultant filtered blood to replace fluid volume and valuable electrolytes. This is then returned to the patient. Thus, in the case of ultrafiltration, a key aspect resides in separating the urea from the other components in the ultrafiltrate such as salts which have also passed through the membrane but which must be reincorporated into the blood in order to maintain the electrolyte composition thereof substantially constant.

A combinations of the two systems described above has also been proposed. Shettigar and Reul (Artif. Organs (1982) 6:17-22), for instance, disclose a system for simultaneous filtration of blood using a hemofilter and dialysis against its purified filtrate, wherein the filtrate is purified by a multi-adsorption system consisting of charcoal for removal of urea and a cation exchanger.

Intermediate systems, i.e. systems that perform no ultrafiltration, yet which adsorb toxic substances directly from the blood have also been proposed. US 2004/0147900 discloses a cartridge for treating medical or biological fluid, in particular blood, consisting of a compartmentalized container, wherein each compartment contains adsorbing particles. The adsorption materials proposed are essentially those disclosed in US 2003/0097086 described above, and thus may effectively remove urea from blood.

As noted above, the adsorbent for regenerating the dialysate is usually activated carbon. However other adsorbents have been proposed for the removal of substances from dialysis fluids or ultrafiltrate. US 3,874,907, for instance, discloses microcapsules consisting essentially of a crosslinked polymer containing sulphonic acid groups and coated with a polymer containing quaternary ammonium groups, for use in an artificial kidney. Examples of the sulphonated polymer include sulphonated styrene/divinyl benzene copolymer and examples of the coating polymer include those obtained by polymerization of for instance vinyldimethylamine monomers. Shimizu et al. (Nippon Kagaku Kaishi (1985), (6), 1278-84) described a chemisorbent composition for the removal of urea from dialysis fluid or hemofiltrate for use in an artificial kidney. The chemisorbent is based on dialdehyde starch (DAS)-urease conjugates and 4,4'-diamidinodiphenylmethane (DADPM).

In conclusion, the prior art discloses both dialysing and ultrafiltration devises, wherein various substances may be used as sorbents.

The problem with the system of the prior art is that however, that they are still too large due to limited sorption capacity of the materials, or not efficient or both in order to allow small, desk-top sized or wearable dialysing and ultrafiltration systems.

It is an object of the present invention to overcome the problems associated with the devices of the prior art and to provide a compact and efficient sorption-filter system for use in hemodialysis and peritoneal dialysis systems and in an artificial kidney.

This problem is solved according to the invention by providing a small sorption-filter device for the removal of toxic substances from hemodialysis and peritoneal fluids, allowing very little dialysate volume and thereby allowing a small, desk-top size or wearable hemodialysis or peritoneal dialysis system. This problem can also be solved according to the invention by providing a small device for the removal of toxic substances directly from the blood from a patient, comprising of a hemofilter for separating patient blood plasma from patient blood cells and a sorption-filter for removing ionic solutes, small-sized molecules and middle-sized molecules from the patient's blood plasma.

Said sorption-filter comprises an absorbing, adsorption, ion-exchange and/or surface crystallisation material with very small particles, preferably nano sized particles having a large specific surface area like the mineral group smectites, other nanoclays, layered double hydroxides, nanoporous silica's, nanoporous or layered alumina silicates (like zeolites), nanosized and /or activated graphite, cyclo-dextrines, crystallisation seeds, a modified biopolymer or combinations thereof.

The biopolymer is preferably a polysaccharide. Examples of polysaccharides include a-glucans having 1,3-, 1,4- and/or 1,6-linkages. Among these, the "starch family", including amylose, amylopectin and dextrins, is especially preferred, but pullulan, elsinan, reuteran and other α-glucans, are also suitable, although the proportion of 1,6-linkages is preferably below 70%, more preferably below 60%. Other suitable polysaccharides include β-1,4-glucans (cellulose), β-1,3-glucans, xyloglucans, gluco-mannans, galactans and galactomannans (guar and locust bean gum), other gums including heterogeneous gums like xanthan, ghatti, carrageenans, alginates, pectin, β-2,1- and β-2,6-fructans (inulin and levan), etc.

### Legends to the Figures

Fig. 1. Cross-sectional presentation of a device according to the invention.
Fig. 2. Opened out 3D-model of a device according to the invention.
Fig. 3. Artist impression of an assembled device according to the invention.

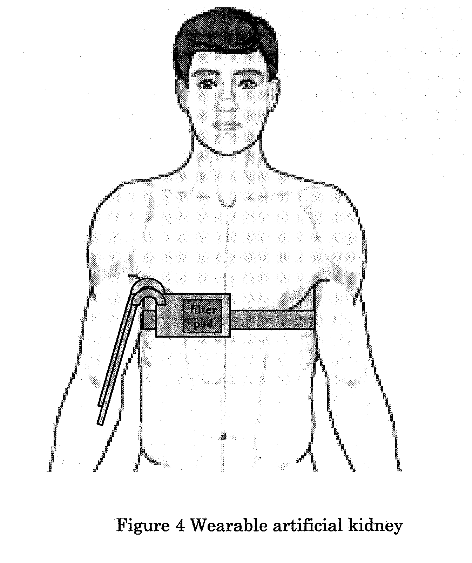

Fig. 4. Shows an example of a wearable artificial kidney system.

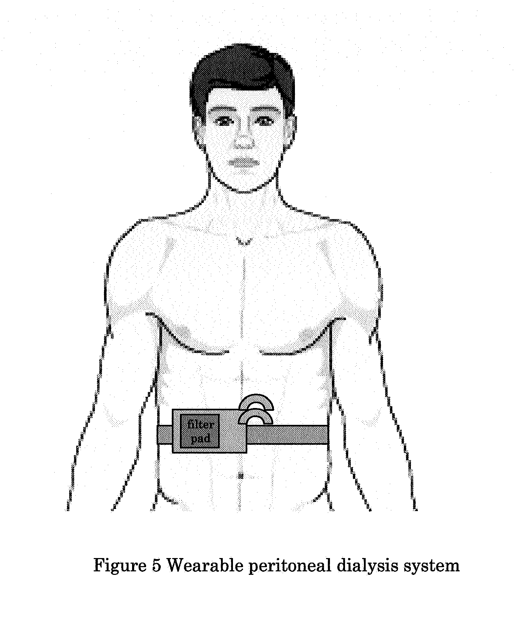

Fig. 5. Shows an example of a wearable peritoneal dialysis system.

The term "sorption" as used herein, refers to both adsorption and absorption. Adsorption is a process that occurs when a gas or liquid or solute (called adsorbate) accumulates on the surface of a solid or more rarely a liquid (adsorbent), forming a molecular or atomic film (adsorbate). It is different from absorption, where a substance diffuses into a liquid or solid to form a "solution". The term sorption encompasses both processes, while desorption is the reverse process.

The term "small-sized molecules", as used herein, refers to molecules with a molecular weight lower than 500 Da, such as uric acid, urea, guanidine, ADMA, creatinine.

The term " middle-sized molecules", as used herein, refers to molecules with a molecular weight between 500 Da and 5000 Da, such as end products from peptides and lipids, amines, amino acids, protein bound compounds, cytokines, leptins, microglobulins and some hormones.

The term "ionic solutes", as used herein, refers to components such as phosphates, sulphates, carbon hydrates, chlorides, ammonia, potassium, calcium, sodium.

"Nano sized" as used herein, refers to a size of approximately 1-1000 nm, more preferably 1-100 nm.

A device of the present invention can take the form of a sorption-filter package that is placed in the dialysis fluid system of a hemodialysis or peritoneal dialysis system enabling the removal of toxins from the dialysis fluid. The sorption filter continuously purifies the dialysate fluid, keeping the the toxin concentration in the dialysis fluid low, resulting in an improvement of the hemodialysis and peritoneal dialysis efficiency, typically with 100%, and reduces the consumption of dialysis fluid needed dramatically, ideally down to 1-10 litres. An additional and optional function of the sorption filter is to release ingredients for supplementing of the blood such as calcium, vitamin A, anti-coagulation agents, anti microbial agents, minerals, specific medicaments etc. This option will simplify the operation of existing hemodialysis and peritoneal dialysis systems and will reduce the chance on occurring infections in the peritoneal dialysis system.

A device of the present invention can take the form of wearable peritoneal dialysis system wherein the sorption-filter package is placed in a wearable peritoneal dialysis system. Due to the continuous filtering of the sorption filter, the volume of dialysate fluid can be reduced to typically 1-2 litres. The wearable peritoneal dialysis device comprises a tubular access system to the abdominal cavity and a unit comprising a fluid pump, power, sensors, electronic control, a facility to place and replace said sorption-filter package, typically on a daily basis and a system to dispose off excess water. An additional and optional function of the sorption filter is to release ingredients for supplementing the blood, such as calcium, vitamin A, anti-coagulation agents, anti microbial agents, minerals, specific medicaments etc. This option will enhance the operation of the peritoneal dialysis system and will reduce the chance on occurring infections.

A device of the present invention can take the form of wearable hemodialysis system wherein the sorption-filter package is placed in a wearable hemodialysis system. Thanks to the continuous filtering of the sorption filter, the volume of dialysate fluid can be reduced to typically 1-2 litres. The wearable hemodialysis device comprises a vascular access tubing system and a unit comprising a small hemofilter system, fluid pump, power, sensors, electronic control, a facility to place and replace said sorption-filter package, typically on a daily basis, and a system to dispose off excess water. An additional and optional function of the sorption filter is to release ingredients for supplementing the blood such as calcium, vitamin A, anti-coagulation agents, anti microbial agents, minerals, specific medicaments etc. This option will simplify the operation of the hemodialysis system and will reduce the chance on occurring infections.

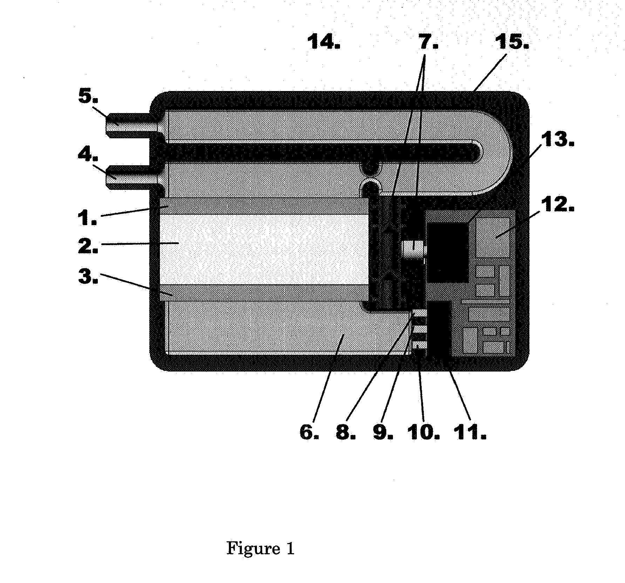

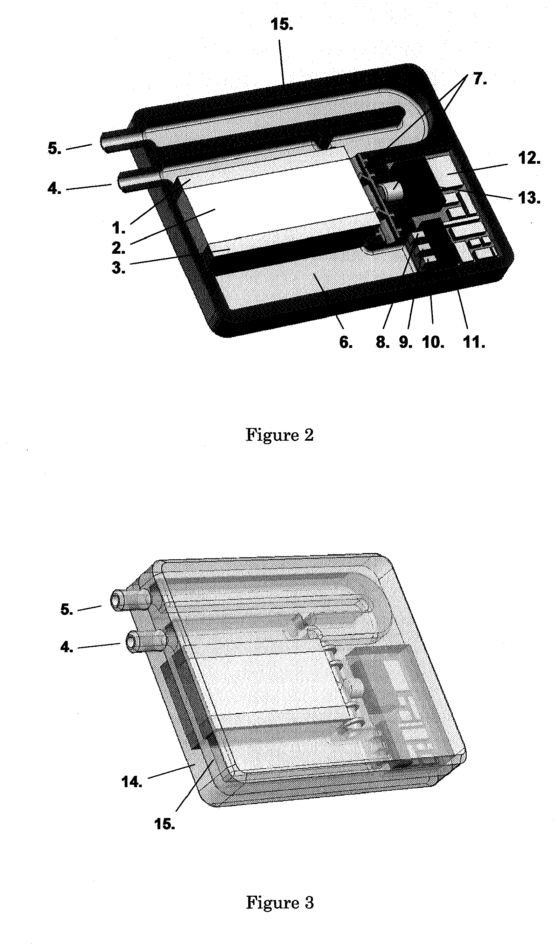

A device of the present invention can take the form of a wearable or desktop sized artificial kidney based on combined ultrafitration and sorption-filtering. It will be understood that in such an embodiment, the device further comprises the necessary tubing, vascular access and feedback systems, pumping, electronics, sensors, power packs and other requirements. However, these are not essential to the present invention. An advantage of the artificial kidney device is that no dialysis fluid will be needed. In a preferred embodiment of this device, the hemofilter and sorption-filter are being combined to form a filter pad, said filter pad comprising an envelop surrounding a filter pad contents, wherein the envelop of said pad comprises said hemofilter, and the contents of said pad comprise said sorbing material. The hemofilter may consist of a material that completely surrounds the sorbing material, or it may form a "window" in the envelop surrounding the sorbing material on the side where the blood is supplied to the filter pad. In other embodiments of a device of the invention the hemofilter comprises an inlet for receiving blood from a patient, and at least one outlet for recovery of patient blood plasma. In addition, the hemofilter may comprise at least a further outlet for recovery of patient blood cells. A representative embodiment of a device of the present invention is depicted in Figures 1, 2 and 3. The device as depicted in Fig. 1 is a cross-sectional presentation of a device, for which an opened-out 3-D model is drawn in Figure 2. Fig. 3 provides an artist impression of an assembled device of Figure 2 showing the various components of Fig. 1 and 2 in transparent housing. Numbering is the same for all parts in the three figures. The device as depicted in Fig. 1 comprises a micron sized hemofilter (1), a sorption filter (2), and a micron sized (hemo) filter (3). These parts (1), (2) and (3) may be provided to form a pad as separate parts or combined into a single part. The skilled person will understand that the filter pad may take various embodiments as described herein above. The device as depicted in Fig. 1 further comprises a blood inlet (4), which may be connected to vascular access and tubing; a blood outlet (5), which is provided for recovery of blood cells and filtered blood plasma; a blood plasma outlet (6) for the recovery of filtered blood plasma flowing out of the sorption filter and bottom hemofilter; a (micro)-pump (7); at least one sensor (8), (9), and/or (10) for measuring salt and/or small and/or middle-sized molecule content, etc.; electronic components (11) (e.g. for digital signal processing (DSP), interfaces for sensors); a RF-wireless radio module (12) providing a link to a medical consultant or computer; a power supply for the device (e.g. incl. power supply for the pump) and accompanying electronics and mechatronic part for the pump (13); a housing, optionally comprised of a monolithic structure, but preferably comprising a first part (14, not drawn) and a second (15) part, which first and second part are separably connected to each other possibly combined with integrated or separate liquid and airtight sealing gaskets to provide the enclosure for the various components of the device, and which, when separated, provide access to the interior of the device. The housing (parts) (14, 15) are suitably injection moulded or otherwise formed plastic housing parts. The sensor(s) (8), (9), and/or (10), and optionally more, may also be located at other positions and can be provided in configurations capable of measuring such parameters as biofilm formation, clothing of blood and microbial contamination. Not drawn in the figure but also comprised in the device is a system to dispose off of excess water. This can take the shape of a simple drain (valve under the filter package) connected to a tube, container, a fluid absorbing package etc. It will be understood that the device of the present invention may take various shapes and the various components may take various positions relative to each other. In fact, the skilled person is free in the design of the device and the location of the various components as long as functionality will be maintained.

The hemofilter in a device of the present invention is preferably a commercially available hemofilter (e.g. such as produced by Membrana GmbH, Wuppertal, Germany) which provides only unselective transport of blood plasma, and has a wide distribution of pore sizes (2 microns and larger).

In an alternative embodiment the sorption-filter in a device of the invention may comprise an inlet for receiving patient blood plasma exiting said hemofilter, and at least one outlet for recovery of purified blood plasma.

A device of the present invention may, in any embodiment, further comprise means for supplementing the (purified) blood plasma or dialysate fluid with at least one substance selected from the group consisting of vitamins such as vitamin C; minerals such as calcium, sodium and potassium; anticoagulants; anti microbial agents and other medicaments.

A device of the present invention may, in any embodiment, further comprise means for selective sorption of middle molecules, vitamins and minerals such as calcium, sodium and potassium. The sorption materials as described (smectites, nanoclay, LDH's, crystallisation seeds, modified biopolymers etc.) are therefore loaded with a certain amount of minerals, vitamins and can only absorb a designated amount.

A device of the present invention may, in any embodiment, further comprise means for selective sorption of middle molecules, vitamins and minerals such as calcium, sodium and potassium in the sorption pad via osmotic differences between blood and dialysate fluid in combination with the filterpad. The blood plasma in this variant essentially remains in circulation and does not enter as a whole the filter pad, but is being cleaned from toxic substances and is being supplemented with nutrients and other vital substances via the dialysate fluid in combination with the sorption unit.

Optionally, the device or filter pad may comprise ion exchange systems.

In another aspect, the present invention provides a method for removing toxic substances from blood, comprising using a device according to the present invention.

In another aspect, the present invention provides a method for removing toxic substances from hemodialysis or peritoneal dialysis fluids, comprising using a device according to the present invention.

The sorption filter in a device of the present invention may take the form of a filter pad, consisting of a rigid or flexible container comprising the sorbing materials.

Thus, in another aspect, the present invention provides a filter pad for separating blood into plasma and cells and for purifying the plasma thus obtained, said filter pad comprising an envelop surrounding a filter pad contents, wherein the envelop of said pad comprises a hemofilter, and the contents of said pad comprise sorbing material for extracting small and middle-sized molecules from said plasma. In a preferred embodiment, the sorbing material is a nanoclay, a layered double hydroxide, a modified biopolymer or a combination thereof.

The sorbing materials are preferably functionalized, such as to exhibit improved sorbing properties of toxic substances such as urea as compared to the non-functionalized material. Preferably, the sorbing materials are nanomaterials, meaning that the material consists of particles or contains particles with a size of preferably 100 nanometres or less in order create a large specific surface area. The nanomaterials will enable very high sorption efficiency and therefore enable a small sized, lightweight and wearable device.

A suitable sorbing material with ion-exchange and/or surface nucleating activity is a clay, preferably a nanoclay. The clay material may be of a natural or synthetic nature. Preferably, the clay has a large contact surface. Very suitable are clay types based on layered silicates, such as layered phyllosilicate composed of magnesium and/or aluminum silicate layers which are each about 7-12 Å in thickness. Especially preferred are smectite-like clay minerals, such as montmorillonite, saponite, hectorite, fluorohectorite, beidellite, nontronite, vermiculite, halloysite and stevensite. These materials impart very favorable mechanical properties. Preferred are smectite, hectorite, saponite, and montmorillonite.

In general, suitable sorbing materials with ion-exchange and/or surface nucleation activity are double layer hydroxides or layered double hydroxides. Layered double hydroxides (LDH's) are a class of ionic lamellar solids with positively charged layers with two kinds of metallic cations and exchangeable hydrated gallery anions. They are also referred to as anionic clays in comparison with cationic clays and also as hydrotalcite-like compounds in the name of the polytypes of the corresponding [Mg-Al] based mineral. LDH's may have different cations, such as Mg, Mn, Fe, Co, Ni, Cu and/or Zn as divalent cations, and Al, Mn, Fe, Co, Ni, Cr and/or Ga as trivalent cations.

In general, particularly suitable sorbing material with ion-exchange and/or surface nucleation activity is a hydrotalcite. Hydrotalcite is an LDH an anionic clay comprised of a mixture of magnesium and aluminium hydroxide and forms a layered, anion intercalation compound. It is generally utilized as acid adsorbent, catalyst carrier, and anion exchanger.

Alternatively, suitable sorbing materials are small particles, preferably nano or micron sized, that induce surface crystallisation by mimicking the morphology of the chemical species to be removed such as urea crystals or urea coated particles. These heterogenous seeds are generally used in filter assisted crystallisation processes. The sorbing process is based on induction of crystallisation of the species to be removed and subsequent filtering of the crystallites.

Other suitable sorbing materials are (modified) biopolymers such as cationic or anionic modified starches. Examples of suitable (modified) starches that can be modified are corn-starch, potato-starch, pee-starch, rice-starch, tapioca starch, banana starch, and manioc starch.

Thus, the sorbing material may comprise of functionalized inorganic and/or polymeric materials.

Optionally, the device may further comprise ionexchange systems or intergallery or intergalleries filtering. The intergalleries are the spaces between the single crystals of the minerals. Preferably, ion exchange relates to anion exchange and/or cation exchange, wherein anions such as Cl⁻, Br⁻, I⁻, SO₄²⁻, PO₃³⁻, NO₃⁻, CO₃²⁻, RCOO- etc., and cations such as K⁺, Ca²⁺, Na⁺, Cu²⁺, Zn²⁺, Al³⁺, NH₃⁺, RₓNH_{y}⁺ etc.

The ion exchange system can be incorporated at any stage in the device, but preferably it is incorporated in the sorption-filter system.

The system of the present invention is i.a. distinguished from the prior art devices in that it makes use of nanomaterials to allow small dimensions for wearability and to allow multiple sorbent stages for removing multiple components (i.e. multiple substances from the blood), both from cationic as well as anionic nature.

The system of the present invention is also distinguished from the prior art devices in that it first separates the blood into plasma and cells/platelets. This plasma is then passed through the sorption filter where one or more additional functions are performed. Thus, a system of the present invention is capable of performing a number of functions. First, it separates blood in to plasma and cells/platelets. In addition, it absorbs the "middle molecules", such as urea, from the blood plasma via the sorption filter. Moreover, in preferred embodiments, by incorporating specific functions in the filter or other part of the device, the system is capable of performing selective sorption of viable minerals on the above mentioned sorbing materials, for instance such as to balance the mineral composition in blood. In still further embodiments, the system is capable of supplying specific components to the blood plasma. These components may be supplied from separate containers or, and preferably, they may be supplied via the incorporation of slow release systems, which are per se known to the skilled person, in the sorption filter system. The additional components may for instance include such ingredients as calcium, vitamin A, anti-coagulation agents, anti microbial agents, minerals, specific medicaments etc.

In a preferred embodiment, the device of the present invention comprises means for regulating the content of minerals and other substances in the blood plasma via selective sorption and controlled release.

In a preferred embodiment therefore, the device for the removal of toxic substances from blood from a patient, comprises a hemofilter and a sorption-filter, wherein the sorption filter removes small and middle-sized molecules from the blood based on the plasma, and includes such functions as selective sorption, controlled release and anti-microbial control.

In a most preferred embodiment, the hemofilter and sorption-filter are comprised in a single part. For instance, the hemofilter may enclose the sorbing material, so as to form a filter pad, wherein the envelop of the pad is formed by the hemofilter material, and the contents of the pad are formed by the sorbing material. The general design of such pads is well known in coffee-machines, such as the Senseo® system. This type of filtering pad will allow simple inclusion of materials for (slow) release of additional components or additional (an)ion exchange systems. In this form it is referred to herein as a sorption and release system.

To accommodate the fitting of a filtering pad, comprising a hemofilter envelop and an sorbing material content, the device of the invention may suitably comprise a filter pad holder, designed to receive the filtering pad and provide for a fluid-flow from across the filter pad, in particular a blood flow, so that blood reaching the filter pad is separated into cells and plasma, plasma passing through the part of the filter that holds the absorbing material, and purified and optionally supplemented blood plasma exits the filter pad and exits the filter pad holder. Thus, in such an embodiment, the inlet for receiving blood from a patient, and the outlet for recovery of purified blood plasma, are present, while outlet for recovery of patient blood plasma and inlet for receiving patient blood plasma exiting said hemofilter, are absent.

It will be understood that the advantage of such a filter pad is that it forms a disposable and replacement part of the device, and can be replaced by a fresh filter pad, for instance when it has been saturated with toxic substances, or if one or more of the components supplemented to the plasma have run out. The sorption capacity of the filter pad for urea and the middle molecules is typically in the range of 30-70% of its own weight, but can reach 100% for specific molecules.

The device of the present invention can be used for filtering or purification of blood of patients with a (developing) renal failure. In a preferred embodiment, the device takes the form of a wearable artificial kidney device, but can also be embodied in desktop sized equipment or in adapted hemodialysis or peritoneal dialysis equipment.

The artificial kidney is able to perform some of the functions which normally will be done by a properly functioning human or animal kidney, in particular filtering of blood and regulation and control of the content of substances in the blood. The device of the present invention comprises an sorption system for capturing toxic substances from the blood and optionally a release system for releasing minerals, vitamins or other substances to the blood, a filter for separating blood cells from blood plasma on the basis of hemofilter having micron-sized pores (min. 2 microns up to 20-30 microns) a vascular access and feedback system for attachment to a body, sensors (connection of filter pad, flow, temperature, anti-coagulation, ion-selective or small and middle-sized molecule selective sensors), a (micro)fluidic circuitry including flow restrictions, pump(s) and tubing, a preferably wireless uplink (wireless radio sensor) to a computer of the care giver or doctor located elsewhere (ZigBee, DECT, GSM/GPRS, Bluetooth or other), an anti-microbial control system consisting of embodied anti-microbial materials in the disposable parts of the system (tubing, vascular access, filter cartridge), a basic user interface for the patient to indicate proper working of the devices, a battery and a PCB with a DSP, inputs and outputs to the sensors, user interface, an integrated blood pressure sensor in the device etc.

The sorption and release system has a temporary use till it reaches its maximum capacity. The content of the sorption and release system can be customized to the individual patient needs.

## Claims

1. A device for the removal of toxic substances from dialysate fluid, blood or bloodplasma, comprising a sorption-filter device for removing toxins, small and middle-sized molecules from the dialysate fluid, blood or bloodplasma, said sorption filter comprising as a sorbing material a smectite, nanoclay, a layered double hydroxide, a modified biopolymer or a combination thereof.

2. Device according to claim1, wherein said device further comprises means for supplementing said dialysate fluid and/or said (purified) blood plasma with at least one substance selected from the group consisting of vitamins, minerals, anticoagulants, anti microbial agents and other medicaments.

3. Device according to claim 1 and 2 wherein said sorption filter is placed in a hemodialysis or peritoneal dialysis system in order to improve the absorption capacity of the dialysis fluid, to mimimize the dialysate fluid volume, to minimize the dimensions of the dialysate system, thereby allowing the dialysate system to be wearable.

4. Device according to claim 3 wherein said dialysis system is a wearable dialysis system.

5. Device according to claim 1 and 2 wherein said sorption-filter is combined with a hemofilter system in order to form an artifical kidney device for the removal of toxic substances from the blood from a patient, comprising a hemofilter for separating patient blood plasma from patient blood cells, and a sorption-filter for removing toxins, small and middle-sized molecules from the patient blood plasma and optionally to supplement the blood with at least one substance selected from the group consisting of vitamins, minerals, anticoagulants, anti microbial agents and other medicaments.

6. Device according to claim 5 wherein said artificial kidney system is a wearable device.

7. Device according to claim 5, wherein said hemofilter comprises an inlet (4) for receiving blood from a patient, and at least one outlet for recovery of patient blood plasma.

8. Device according to claim 5, wherein said hemofilter comprises at least a further outlet for recovery of patient blood cells.

9. Device according to any one of the preceding claims, wherein said sorption-filter comprises an inlet for receiving patient blood plasma exiting said hemofilter, and at least one outlet for recovery of purified blood plasma.

10. Device according to claim 3, wherein said sorption-filter is combined with a permeable envelop to form a filter pad comprising an envelop surrounding a filter pad contents, wherein the envelop of said pad comprises a permeable membrane and the contents of said pad comprise said sorbing material.

11. Device according to claim 5, wherein said hemofilter and said sorption-filter are combined to form a filter pad comprising an envelop surrounding a filter pad contents, wherein the envelop of said pad comprises said hemofilter, and the contents of said pad comprise said sorbing material.

12. Device according to any one of the preceding claims, wherein said device further comprises ion exchange systems.

13. Device according to any one of the preceding claims, wherein said device further comprises surface crystallisation systems.

14. Method for removing toxic substances from blood, comprising using a device according to any one of claim 1-13.

15. A filter pad for separating blood into plasma and cells and for purifying the plasma thus obtained, said filter pad comprising an envelop surrounding a filter pad contents, wherein the envelop of said pad comprises a hemofilter, and the contents of said pad comprise sorbing material for extracting urea and small and middle molecules from said plasma.

16. Filter pad according to claim 15, wherein said sorbing material is a smectite, a nanoclay, a layered double hydroxide, a surface crystallisation seed, a modified biopolymer or a combination thereof.

17. Filter pad according to claim 16 or 17, wherein said filter pad further comprises means for supplementing dialysate fluids and/or blood plasma with at least one substance selected from the group consisting of vitamins, minerals, anticoagulants, anti microbial agents and other medicaments.
